# EUROPEAN PATENT SPECIFICATION

(11) **EP 3 538 897 B1**
(45) Date of publication and mention of the grant of the patent: **28.08.2024**
(21) Application number: 16798259.4
(22) Date of filing: 10.11.2016
(51) Int. Cl.: G01N 33/84, G01N 31/22, G01N 33/18, G01N 21/3577, G01N 21/78

(54) **MOLYBDENUM BLUE ASSAY FOR MEASUREMENT OF CHEMICAL SPECIES**
TEST ZUR BESTIMMUNG VON CHEMISCHEN SPECIES MITTELS MOLYBDENUM BLUE
PROCEDE DE DETECTION BASE SUR LE BLEU DE MOLYBDENE

(43) Date of publication of application: 18.09.2019
(73) Proprietor: National Oceanography Centre, Southhampton SO14 3ZH (GB)
(72) Inventor: LOUCAIDES, Socratis, Southampton Hampshire SO14 3ZH (GB); CLINTON-BAILEY, Geraldine, Southampton Hampshire SO14 3ZH (GB); MOWLEM, Matthew, Southampton Hampshire SO14 3ZH (GB)
(74) Representative: Barker Brettell LLP
(86) International application number: PCT/GB2016/053533
(87) International publication number: WO 2018/087507

(56) References cited:
- WO-A1-85/04721
- US-A- 4 009 004
- US-A- 5 858 797
- NAGUL EDWARD A ET AL: "The molybdenum blue reaction for the determination of orthophosphate revisited: Opening the black box", ANALYTICA CHIMICA ACTA, vol. 890, 10 August 2015 (2015-08-10) - 19 July 2015 (2015-07-19), pages 60 - 82, XP029274791, ISSN: 0003-2670, DOI: 10.1016/J.ACA.2015.07.030
- LEGIRET FRANÇOIS-ERIC ET AL: "A high performance microfluidic analyser for phosphate measurements in marine waters using the vanadomolybdate method", TALANTA, vol. 116, 2013, pages 382 - 387, XP028743879, ISSN: 0039-9140, DOI: 10.1016/J.TALANTA.2013.05.004

## Description

The present invention relates to the sensing and measurement of chemical species in water i.e. an assay. More particularly, it relates to a process for measurement (including continuous monitoring or periodic measurement) of chemical species, such as phosphates, arsenates, and germanates, within a body of water over a period of time.

Water is a precious natural resource. In order to help preserve, protect and/or manage this resource effectively, it is important to be able to understand and monitor it, e.g. by obtaining reliable information on water quality and content. For instance, it may be desirable to sense and measure chemical species within a body of water such as an ocean, a sea, an estuary, a river, a reservoir, a lake or a pond or in agricultural water, waste water, drinking water, aquaculture and industrial water, since changes in concentration may indicate changes in water quality (including suitability for use in a defined process) and/or in the prevailing environmental conditions for plants and animals that live in the water.

Phosphate is a key nutrient supporting primary production in the ocean. In most of the open ocean phosphate concentrations can be well below 0.1 µM and therefore, below the limit of detection of standard nutrient analyzers. Although more sensitive nutrient analyzers exist, they are bulky and complex instruments only appropriate for ship-based expeditions. Therefore, high resolution low concentration measurements over extended periods of time at moderate cost are currently not possible.

The standard method for determination of phosphate in sea water is the so-called "molybdenum blue" assay. A sea water sample is mixed with an acid solution of molybdenum having oxidation state +6 (Mo^{VI}), for example ammonium molybdate, to produce PMo₁₂O₄₀³⁻ anion (alpha Keggin structure), which gives a yellow solution. The anion is then reduced to form PMo₁₂O₄₀⁷⁻ anion (a mixed valence complex having a beta Keggin structure), which gives a blue solution. The amount of the blue coloured anion produced is proportional to the amount of phosphate present and the absorption can be measured using a colorimeter to determine the amount of phosphorus.

The standard assay suffers from various drawbacks. The blue solution is unstable and forms a precipitate (the reduced phosphomolybdic (PMB) acid complex) within a few hours of formation. Therefore the blue solution must be analysed soon after its preparation, which is not always practical if in the field, for example if the reagent is added to discrete samples in the field for later laboratory analysis. Moreover the assay can be sensitive to temperature and salinity. This is particularly difficult when sampling oceanic water columns since the water is saline and the temperature can change rapidly.

US5858797A discloses a unitized, one step test reagent composition, device and method for the determination of phosphorus in various test sample fluids such as soil extracts.

The scope of the invention is defined by the claims. Claim 1 defines a process for measuring a concentration of a chemical species in an aqueous sample comprising:
providing an aqueous sample containing the chemical species, the sample being obtained from industrial water or agricultural water or waste water or water for aquaculture or a body of water selected from an ocean, a sea, an estuary, a river, a reservoir, a lake, a ditch or a pond;
mixing the aqueous sample with a first reagent comprising a solution of a molybdenum VI salt to provide a first solution;
mixing the first solution with a second reagent comprising a reducing agent to form a second solution; and
measuring a property of the second solution to determine the concentration of the chemical species;
wherein polyvinylpyrrolidone is added to the aqueous sample, the first reagent, the second reagent, the first solution and/or the second solution such that the second solution comprises no more than 0.5% (w/v) polyvinylpyrrolidone.

It will be understood that the first and second solutions may correspond to the yellow and blue solutions respectively in the conventional molybdenum blue assay. In which case, the intensity of the blue solution is measured.

Polyvinylpyrrolidone (PVP) is a water-soluble polymer made from the monomer N-vinylpyrrolidone. As far as the inventors are aware, PVP has never been employed to assay agricultural, natural or industrial water. PVP has been shown to be surprisingly effective, even as compared to anionic surfactants. The benefit is not observed with all polymers; some polymers, such as PVA, completely suppress the reaction.

The process of the present invention has been found to have advantages over the standard molybdenum blue assay, as demonstrated by the examples. Embodiments of the invention may have one or more of the following benefits relative to the standard:
Decreased temperature effects, e.g. a wider operating temperature range, reduced temperature effects on the kinetics (rate) of the reaction, increased stability of the PMB complex at low temperatures;
Increased sensitivity (at least 3 fold in some embodiments);
Increased colour stability of the second solution;
Decreased effects of common interferences (e.g. silicate); and
Increased precision and accuracy.

If the prior art assay is employed without a surfactant, carrier or stabilising agent the sensitivity is reduced and precipitation may occur. Precipitation is problematic in channel based flow analysers since it may block the channel. Further, the PMB acid complex adsorbs to surfaces without such an additive.

In embodiments the PVP may have molar (molecular) mass of at least 2500, 5000 or 8000g/mol and / or no more than 2500000, 1000000 or 100000g/mol. In embodiments the PVP has a molecular mass of approximately 10,000g/mol.

PVP is added during the process such that it is present in the second solution, such that the second solution comprises no more than 0.5% (w/v) polyvinylpyrrolidone. The PVP can be added directly to the second solution or alternatively, it can be added to the sample, the first reagent, the first solution and / or the second reagent. For practical reasons, it may be convenient for the PVP to be present in the first reagent and / or the second reagent. In this way, a stock solution can be prepared having a known concentration and small quantities of PVP can be delivered accurately.

### Second solution

The second solution may be a blue solution. The second solution comprises PVP, the first reagent, the second reagent and the aqueous sample containing the chemical species to be analysed.

The second solution comprises no more than 0.5% (w/v) polyvinylpyrrolidone. In a series of embodiments the second solution comprises at least 0.0001%, at least 0.001%, at least 0.005%, at least 0.01%, at least 0.05%, or at least 0.1% PVP and/or no more than 0.1%, no more than 0.05%, no more than 0.01%, no more than 0.005%, no more than 0.001% or no more than 0.0005% PVP. In a particular embodiment the second solution comprises from 0.001% to 0.05% PVP. All percentages are weight by volume (w/v).

In a series of embodiments the second solution comprises at least 0.0001%, at least 0.001%, at least 0.005%, at least 0.01%, at least 0.05%, or at least 0.1% molybdenum salt and/or no more than 0.5%, no more than 0.1%, no more than 0.05%, no more than 0.01%, no more than 0.005%, no more than 0.001% or no more than 0.0005% molybdenum salt. In a particular embodiment the second solution comprises from 0.001% to 0.05% molybdenum salt. All percentages are weight by volume (w/v). It will be understood that these values depend on the amount of Mo^{VI} salt that was present in the first reagent.

The amounts of PVP and molybdenum VI salt employed in the process can be compared. In embodiments the mass of PVP is equivalent to at least 5, 10, 20, 50 or 100% of the mass of the molybdenum VI salt and / or no more than 300, 200, 150 or 100% of the molybdenum VI salt. In one embodiment the mass of PVP corresponds to 10 to 150% of the mass of the molybdenum VI salt.

In a series of embodiments the second solution comprises at least 0.001%, at least 0.01%, at least 0.05%, at least 0.1%, at least 0.5%, or at least 1% reducing agent and/or no more than 5%, no more than 1%, no more than 0.5%, no more than 0.1%, no more than 0.05%, no more than 0.01% or no more than 0.005% reducing agent. In a particular embodiment the second solution comprises from 0.1% to 0.5% reducing agent. All percentages are weight by volume (w/v). It will be understood that these values depend on the amount of reducing agent that was present in the second reagent.

The amounts of PVP and the reducing agent employed in the process can be compared. In embodiments the mass of PVP is equivalent to at least 0.01, 0.05, 0.1 or 0.5% of the mass of the reducing agent and / or no more than 100, 50, 10 or 5% of the reducing agent. In one embodiment the mass of PVP corresponds to 0.5 to 10% of the mass of the reducing agent.

The second solution has a volume equal to the sum of the volumes of the first reagent, the second reagent and the aqueous sample. The H⁺ to Mo ratio can be determined in the blue solution and we have found a ratio of approximately 72:1 to be most successful. In embodiments the second solution has a ratio of H⁺ : Mo of at least 50:1 or at least 70:1 and / or no more than 300:1, no more than 260:1, or no more than 100:1.

The chemical species may be known as the analyte or the component which is of interest in the assay. In embodiments the chemical species is selected from the group consisting of phosphate, arsenate and germanate. The chemical species is measured colorimetrically at wavelengths from 400 - 900nm (the exact wavelength is analyte dependent).

Beer's Law (or Beer-Lambert-Bouguer Law) is used to determine the concentration of an analyte in solution based on the absorption or transmission of light at particular wavelengths. The intensity of the colour in solution is proportional to the concentration of the analyte of interest. The concentration is calculated generally through standardisation (i.e. comparison between the absorption or transmission of a blank and a standard series of known concentrations). A certified reference material is often used to confirm the precision and accuracy of the technique.

In embodiments measuring the property of the second solution comprises measuring the intensity of light of a given wavelength that is transmitted and/or absorbed.

In embodiments the intensity of light that is transmitted or absorbed by the second solution is measured using a colorimeter or a spectrophotometer.

The assay of the invention has been found to be especially useful for detecting phosphate and arsenate. The standard molybdenum assay is employed to measure silicate but the inventors have discovered that the use of PVP suppresses the silicate signal. This has the advantage that other chemical species can be detected more easily because silicate generally occurs at much high concentrations in the environment.

In embodiments the chemical species is phosphate (PO₄³⁻). Phosphates play a vital role in animal and plant life. Adenosine triphosphate (ATP) transports energy within cells for metabolism. Phosphate generates a blue solution having a peak absorption signal at 880 nm, with a secondary absorption peak at 700 nm. The second peak (at 700 nm) is most frequently used when longer measurement cells are employed (> 1cm). This is because the 880 nm peak is near the water absorption peak in the near IR (~1000nm).

In embodiments the chemical species is an arsenate (AsO₄³⁻). Arsenic is poisonous, so it is important for health that is kept at low concentration. Arsenate generates a blue solution having a peak absorption signal at 840 nm and a secondary absorption peak between 600-700nm.

In embodiments the chemical species is germanate (GeO₄⁴⁻). Germanium is an important biogeochemical tracer in marine science. Germanate generates a blue solution having a peak absorption signal at 825 nm.

In embodiments the property of the second solution is measured at least one day, at least two days, at least three days or at least a week after the second solution is formed. This is not possible using the standard assay since the blue solution is unstable. Hence, the water sample may be mixed with the first and second reagents in the field and then analysed later in the laboratory. This is beneficial since field sample storage can be problematic, for example such as bacterial degradation.

In embodiments the assay is employed in an analyser (e.g. micro-fluidic sensor, stopped flow analyser, segmented flow analyser, flow injection analyser, continuous flow analyser) where the intensity of the second solution is measured at least every 0.1s, at least every 1s, at least every 10s, at least every 100s, at least every 1000s and at least every 10000s, at least every one day, at least two days, at least three days or at least a week.

In embodiments the property of the second solution is measured no more than four weeks or no more than two weeks after the second solution is formed.

### Aqueous sample

In embodiments the aqueous sample has a temperature of at least 0, 1, 2, 3, 4, 5 or 10°C and/or a temperature of no more than 50, 40, 30 or 20°C. A sample taken from the bottom of the ocean may have a low temperature e.g. 2°C whereas a sample from a tropical location would have a higher temperature e.g. 30°C. The standard molybdenum blue assay is temperature sensitive.

The sample may comprise components which confer an "anti-freeze" effect, that keeps the sample liquid below 0°C. PVP , glyerol, and / or ethylene glycol might be considered to confer such an effect in some circumstances. Hence, in some embodiments the sample, first reagent, second reagent, first solution or second solution may have a temperature of at least -20°C.

In embodiments the aqueous sample is saline (contains NaCl). The process of the invention is particularly useful for seawater. Without being bound by theory, the inventors propose that the addition of PVP is especially beneficial in water having a higher ionic strength.

### First reagent

In embodiments the PVP is added to the first reagent, such that the second solution comprises no more than 0.5% (w/v) polyvinylpyrrolidone.

In embodiments, the first reagent comprises PVP. In a series of embodiments the first reagent comprises at least 0.0001%, at least 0.001%, at least 0.005%, at least 0.01%, at least 0.05%, at least 0.1% or at least 0.5% PVP and/or no more than 1%, no more than 0.5%, no more than 0.1%, no more than 0.05%, no more than 0.01%, no more than 0.005%, no more than 0.001% or no more than 0.0005% PVP. In a particular embodiment the first reagent comprises from 0.005% to 0. 1% PVP All percentages are weight by volume (w/v).

In an alternative embodiment, the first reagent does not comprise PVP. For example, if the PVP is added to the second reagent.

The first reagent comprises a molybdenum VI (Mo^{VI}) salt i.e. molybdenum having oxidation state +6. In embodiments the first reagent comprises at least 0.01, 0.05, 0.1, 0.5 or 1 % Mo^{VI} salt and / or no more than 10, 5, 3, 2 or 1 % Mo^{VI} salt. All percentages are weight by volume (w/v).

The molybdenum VI salt may be any molybdenum salt that is soluble in water. In embodiments the molybdenum VI salt is ammonium molybdate (such as ammonium molybdate tetrahydrate) and / or sodium molybdate. In embodiments the first reagent comprises from 0.05 to 0.1 % ammonium molybdate.

In embodiments the first reagent comprises an acidic solution of molybdenum VI. In some embodiments the first reagent has a pH of no more than 6.9, no more than 4 or no more than 2. In embodiments the first reagent comprises hydrochloric acid (HCl) and / or sulphuric acid (H₂SO₄).

In embodiments the first reagent additionally comprises potassium antimonyl tartrate (PAT). In one such embodiment the first reagent comprises PAT at a concentration up to 10 g L⁻¹ (or 1% w/v). The antimony acts as a catalyst and the tartaric acid inhibits silicon interference.

In one embodiment the first reagent comprises ammonium molybdate, sulphuric acid and PAT.

### Second reagent

In embodiments the PVP is added to the second reagent, such that the second solution comprises no more than 0.5% (w/v) polyvinylpyrrolidone.

In embodiments, the second reagent comprises PVP. In a series of embodiments the second reagent comprises at least 0.0001%, at least 0.001%, at least 0.005%, at least 0.01%, at least 0.05%, at least 0.1% or at least 0.5% PVP and/or no more than 1%, no more than 0.5%, no more than 0.1%, no more than 0.05%, no more than 0.01%, no more than 0.005%, no more than 0.001% or no more than 0.0005% PVP. In a particular embodiment the second reagent comprises from 0.005% to 0.1% PVP. All percentages are weight by volume (w/v). In an alternative embodiment, the second reagent does not comprise PVP.

The second reagent comprises a reducing agent. The reducing agent donates electrons to the heteropolymolybdate complex. In embodiments the reducing agent comprises ascorbic acid or stannous chloride (SnCl₂). Other reducing agents have been trialled, but with limited success. Stannous chloride and ascorbic acid are most widely applied, with the ascorbic acid as the "industry standard" due to its kinetics and non-toxicity.

In embodiments the second reagent comprises at least 0.0001, 0.001, 0.01, 0.1, 1, 10 or 30% ascorbic acid and / or no more than 33, 10 or 1 % ascorbic acid. The solubility limit of ascorbic acid in water at standard ambient temperature and pressure (SATP, 25°C and 100kPa) is approximately 33%, and the inventors have found this to be convenient. All percentages are w/v.

In one embodiment the second reagent comprises ascorbic acid and PVP.

The first reagent, the second reagent and the aqueous sample each has a volume measured at standard ambient temperature and pressure (SATP, 25°C and 100kPa). It will be understood that the volumes of the aqueous sample, the first reagent and the second reagent will be selected depending on the concentration of the components therein. For convenience the ratio of the volumes of the sample : the first reagent : the second reagent may be 1 to 10: 1 to 10 : 1 to 10, or approximately 1:1:1.

The analysis may be carried out conveniently on an analyser that employs small volumes of liquids e.g. a microplate reader, micro-fluidic sensor, stopped flow analyser, segmented flow analyser, flow injection analyser, continuous flow analyser.

In embodiments the aqueous sample has a volume of at least 0.01, 0.1, 1, 5, 10, 50, or 100mL and / or no more than 1000, 500, 200, 100, 50, 10, 5, 3, 1, 0.5 or 0.1mL.

In embodiments the first reagent has a volume of at least 0.01, 0.1, 1, 5, 10, 50 or 100mL and / or no more than 1000, 500, 200, 100, 50, 10, 5, 3, 1, 0.5 or 0.1mL.

In embodiments the second reagent has a volume of at least 0.01, 0.1, 1, 5, 10, 50 or 100mL and / or no more than 1000, 500, 200, 100, 50, 10, 5, 3, 1, 0.5 or 0.1mL.

In embodiments the second solution has a volume of at least 0.03, 0.1, 0.3, 1, 10, 50, 100 or 200mL and / or no more than 3000, 1000, 50, 200, 100, 50, 30, 20, 10, 5, 1 or 0.5mL.

The first solution has a volume equal to the sum of the volumes of the first reagent and the aqueous sample.

In embodiments the process comprises an initial step of obtaining an aqueous sample from a body of water such as an ocean, a sea, an estuary, a river, a reservoir, a lake, a pond, agricultural water, waste water, drinking water, aquaculture and industrial water. In embodiments the aqueous sample comprises sea water and/or freshwater. It will be understood that an aqueous sample obtained from an estuary will comprise both sea water and fresh water.

In embodiments the process can comprise an initial step of filtering the aqueous sample, prior to mixing with the first reagent. The sample may be filtered through a membrane having a maximum pore size of 0.45µm or, a membrane of pore size 0.2 µm
In embodiments the process comprises an initial step of oxidizing the aqueous sample, prior to mixing with the first reagent. The sample may be oxidized using ultraviolet light or by other chemical means. Oxidation is useful for As measurement - more specifically for oxidation of AsIII to As V. This allows total As to be measured using the process of the invention. Oxidation is also useful for organophosphorus as this produces inorganic phosphorus in proportion to the amount of organophosphorus and this inorganic phosphorus can be measured using the process of the invention.

In embodiments the process is carried out using an LOC (lab on a chip) device. An LOC device employs precision fabricated microfluidic chips with pumps and electronics in one package. An LOC device allows the process to be carried out on a small scale. In one such embodiment the aqueous sample has a volume of no more than 1ml. The invention is advantageous in this platform because the high surface area to volume ratios of the fluidic paths can lead to surface interactions with the heteropoly acid (HPA) (via for instance hydrogen bonds with the PMMA). We propose that the PVP has a two-fold function. It acts as a dispersant, and so prevents the precipitation of the reduced HPA (which can lead to blockages in microfluidic systems); and it acts as an electron donor thereby enhancing the reduction process and interacting with the HPA to decrease sorption to the PMMA.

In order that the invention may be well understood, it will now be described by way of example only with reference to the accompanying drawings, in which:
Figure 1 Absorbance scan from 600-900nm @ 5µM phosphate for SDS, SOS and LDS;
Figure 2 Absorbance scan from 600-900nm @ 5µM phosphate for PVP 0.005-0.1%;
Figure 3 Absorbance scan from 600-900nm @ 5µM phosphate for Glycerol, Triton-X and Brij-35;
Figure 4 Absorbance scan from 600-900nm @ 5µM phosphate for surfactant mixtures;
Figure 5 Limit of detection for surfactants trialled. Calculated using 3x standard deviation of 10 blank measurements divided by the slope of a calibration curve from 0-1 µM PO₄;
Figure 6 Comparison of slopes using 2 calibration curves. Graph (a) uses a calibration range from 2.5-7.5 µM PO₄; the bottom graph (b) uses a calibration range from 0-1 µM PO₄;
Figure 7 Limit of detection for ASW and MQ for each of the additives using 0-1 µM PO₄³⁻ calibration;
Figure 8 Change in calibration slope for each of the additives;
Figure 9 Colour formation (absorption units) as measured at the peak absorbance wavelength for each additive.
Figure 10 Calibration for the 41 mm measurement cell at 20°C

### Materials

All chemicals were of analytical-reagent grade or higher, and all solutions were prepared using milli-q (MQ) water (Merck Millipore). The final proportions after mixing with the sample are as described by Patey et al [Patey, M.D., et al., Interferences in the analysis of nanomolar concentrations of nitrate and phosphate in oceanic waters. Analytica Chimica Acta, 2010. 673(2): p. 109-116.].

A stock solution of potassium antimonyl tartrate (PAT) was prepared by mixing 3 g of potassium antimonyl tartrate trihydrate (C₄H₂KO₆Sb·1.5H₂O) in 1 L MQ. The molybdic acid solution (reagent 1) was prepared by mixing 0.575 g ammonium molybdate tetrahydrate ((NH₄)₆Mo₇O₂₄.4H₂O, 1235.86g/mol, Sigma Aldrich) with 24 mL 5 M sulfuric acid (Sigma) and 12.5 mL of the stock PAT solution diluted to 1L with MQ water. The reducing agent (reagent 2) was made by adding 10 g L-ascorbic acid and the surfactant as detailed in Table 1 to 1 L MQ water. Standards were made using sequential dilution from a 1 mM stock solution of potassium dihydrogen orthophosphate (KH₂PO₄, Fisher Scientific) were prepared in either MQ water (for fluvial measurements) or a simplified artificial seawater (ASW) made by mixing 35 g NaCl (Sigma) and 0.5 g NaHCO₃ (Fisher) in 1 L MQ. Blank solutions were either MQ or ASW depending on the deployment environment. Silicate and arsenate solutions were prepared using sodium silicate pentahydrate (Na₂SiO₃·5H₂O, Sigma) and sodium arsenate dibasic heptahydrate (Na₂HAsO₄·7H₂O, Sigma Aldrich) was prepared after Patey et al.

Various additives were investigated alone and in combination as summarised in the table below.

| **Additive** | | **Formula** | **RMM** | **CMC (mM) @RTP, MQ** | **Approxim ate cost (£/g)** | **Concentration added to ascorbic acid, % (w/v)*** |
|---|---|---|---|---|---|---|
| **Anionic surfactant** | | | | | | |
| Sodium dodecyl sulfate (SDS) | | C₁₂H₂₅NaO₄S | 288.37 2 | 8.1[1] | 0.89 | 0.70 |
| Sodium n-octyl sulfate (SOS) | | C₈H₁₇NaO₄S | 232.27 | 130[1] | 26.20 | 9.10 |
| Lithium dodecyl sulfate (LDS) | | CH₃(CH₂)₁₁OSO₃L i | 272.33 | 7-10 | 8.36 | 0.90 |

| **Nonionic surfactant** | | | | | | |
|---|---|---|---|---|---|---|
| Triton X100 | | *t*-Oct-C₆H₄-(OCH₂CH₂)ₓOH, x= 9-10 | Ave 625 | 0.2-0.9 | 5.00/ml | 0.02 |

| **Additive** | | **Formula** | **RMM** | **CMC (mM) @RTP, MQ** | **Approxim ate cost (£/g)** | **Concentration added to ascorbic acid, % (w/v)*** |
|---|---|---|---|---|---|---|
| Brij^{®}35 | | CH₃₍CH₂)₁₀CH₂(O CH₂CH₂)ₙOH | Ave 1,198 | 0.09 | 1.30/ml | 0.03 |

| **Polymer** | | | | | | |
|---|---|---|---|---|---|---|
| PVP | | (C₆H₉NO)ₙ | Ave 10,000 | n/a | 0.20 | 0.005 - 0.1 |
| Glycerol | | | 92.09 | n/a | 0.30/ml | 0.01 |

| **Combinations** | | | | | | |
|---|---|---|---|---|---|---|
| 0.01% PVP plus | | | | | | |
| | • SDS | | | | | 0.70 (SDS) |
| | • LDS | | | | | 0.90 (LDS) |
| | • Brij^{®}35 | | | | | 0.03 (Brij^{®}35) |
| SDS + TritonX | | | | | | 0.70 (SDS) + 0.02 (Triton X100) |

| | | | | | | |
|---|---|---|---|---|---|---|
| *This was above the CMC (critical micelle concentration) at RTP after dilution with the sample and molybdenum reagent | | | | | | |

Comments on the various additives are provided below.

### Sodium dodecyl sulphate (SDS)

SDS is the most commonly used surfactant for the ortho phosphate assay. It is used in commercial instruments (such as the SEAL^{®} Analytical QuAAtro nutrient analyser) and has been used routinely in a number of studies. It has been found to outperform other surfactants in terms of reaction stability and kinetics [Nagul, E.A., et al., The molybdenum blue reaction for the determination of orthophosphate revisited: Opening the black box. Analytica chimica acta, 2015]. There are two major disadvantages to using this surfactant; a) it is toxic and highly flammable and b) it has a high Krafft point (15°C), meaning it will begin to fall out of solution when stored at low temperature. It is generally mixed with the ascorbic acid which requires storing at low temperatures (~4°C) in the dark unless made fresh each day and so is incompatible for long term reagent stability.

### Sodium n-octyl sulfate (SOS)

SOS was investigated as an alternative to SDS to determine if using a shorter chained surfactant was an option for low temperature applications and reagent storage. The biggest disadvantage is the cost of this surfactant and like the SDS, it is toxic and flammable.

### Lithium dodecyl sulfate (LDS)

LDS is used in low temperature biological applications (namely electrophoresis). It has a working temperature range of approximately 4-35°C making it more useful for a wider number of applications, but is also toxic and flammable.

### Triton X100

Triton X100 is also used in commercial instruments (such as the SEAL Analytical QuAAtro analyser). It is also an example of a non-ionic surfactant to provide a comparison.

### Brij^{®}35

Brij^{®}35 is a common non-ionic laboratory surfactant for inorganic element analysis.

### Polyvinylpyrrolidone (PVP)

It is the cheapest of the products being tested, is non-toxic, has complexing stability, is relatively inert and is resistant to thermal degradation. It is commercially available in a number of molecular weights, for instance: 10k, 29k, 40k, 55k, 360k, 1,300k.

### Glycerol

Glycerol is a water soluble lubricant that lowers the freezing point of the solution. It should not interact with the reaction.

Other surfactants that have been trialled with no success using the microfluidic technology are:
1. Tergitol-10; this is a lineating surfactant non-ionic surfactant.
2. Poly-vinyl alcohol
3. Sodium lauryl sulfate
4. Tween 20 and tween 80, which are polyoxyethylene surfactants similar to the Brij 35.

None of these surfactants were carried forward to further trials.

### Microplate experiments

### Methodology

The screening of the surfactants was undertaken using a PIKE Technologies out of compartment microplate reader, attached using a fibre optic cable to an Aglient Cary-60 UV-visible spectrophotometer.

To each well of a 96 well microplate (Greiner, Bio-One UV star) 100 µL of standard solution was added to 100 µL of reagent 1. The yellow colour was left to develop for 15 minutes before adding 100 µL of reagent 2 and allowing the blue colour to develop for 15 minutes at room temperature. The long development time ensures reaction completion for all solutions and allows for any discrepancies in the reduction kinetics of the various surfactants trialled. The absorption at wavelengths 600-900 nm at a resolution of 1 nm was then measured for each well. A standard series made up in with ASW or MQ water was measured for each additive using a concentration range from 0 - 7.5 µM PO4³⁻.

### Results

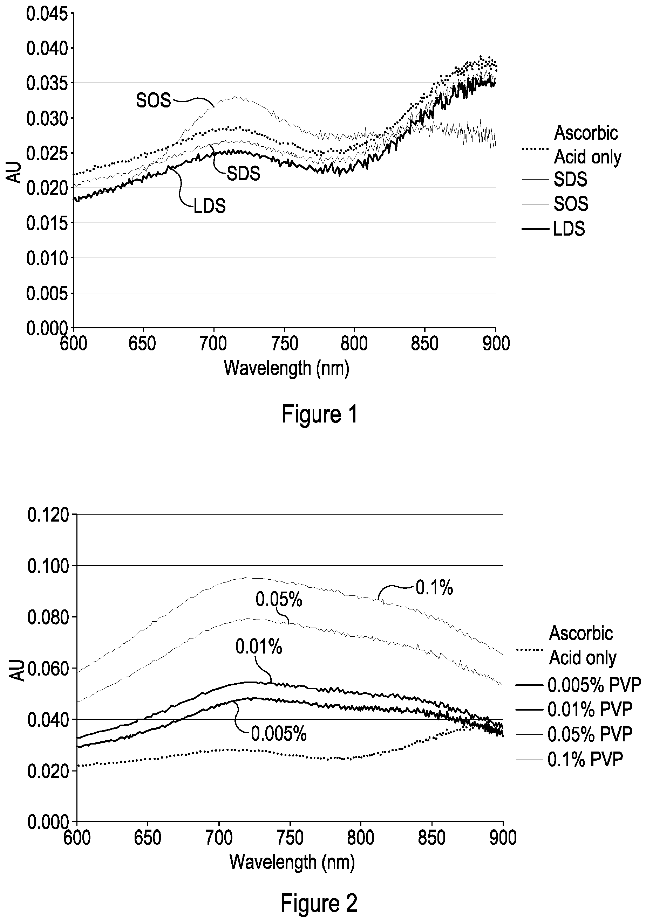

The effect of the additive on the peak absorption wavelength is demonstrated in Figures 1-4. All scans have been blank corrected to remove any interference from water. Referring to figure 1, it can be seen that the anionic surfactants LDS and SDS do not affect the peak wavelengths. However, potentially due to the concentration used, SOS sees a peak enhancement at 700nm and the suppression at 880nm.

Referring to figure 2, the addition of PVP increases the peak height and breadth at ~700 nm, masking the 880 nm peak. The signal is also greatly enhanced. It can be seen that increasing the concentration of PVP from 0.005% to 0.1% increases the intensity. This indicates that the PVP is not a passive component of the reagent.

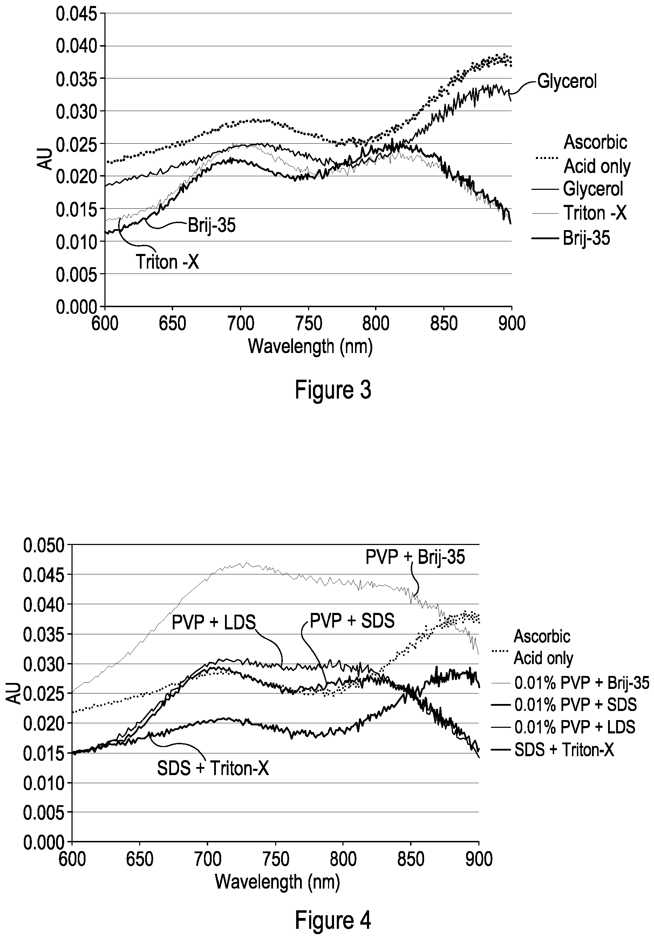

Referring to figure 3, the addition of Triton-X and the Brij-35 both suppress the colour development and absorb at a shorter wavelength than the ascorbic acid alone, while the glycerol does not interact with the reaction.

Referring to figure 4, it can be seen that the combination of 0.01% PVP and Brij^{®}35 enhances the signal relative to ascorbic acid only. The enhancement is comparable to that shown in figure 2 for 0.01% PVP without Brij^{®}35. The combination of SDS and Triton-X suppress the colour development.

The table below details the absorption peaks used in the study.

| **Additive** | **Absorbance peak (nm)** |
|---|---|
| None (ascorbic acid only) | 710, 880 |
| SDS | 710, 880 |
| SOS | 710 |
| LDS | 710, 880 |
| 0.005% PVP | 720 |
| 0.01% PVP | 725 |
| 0.05% PVP | 730 |
| 0.1% PVP | 730 |
| Glycerol | 710, 880 |
| Brij-35 | 700, 810 |
| Triton X | 700, 810 |
| 0.01% PVP + Brij-35 | 725 |
| 0.01% PVP + SDS | 725 |
| 0.01% PVP + LDS | 750 |
| SDS + Triton X | 710, 880 |

### Calibrations

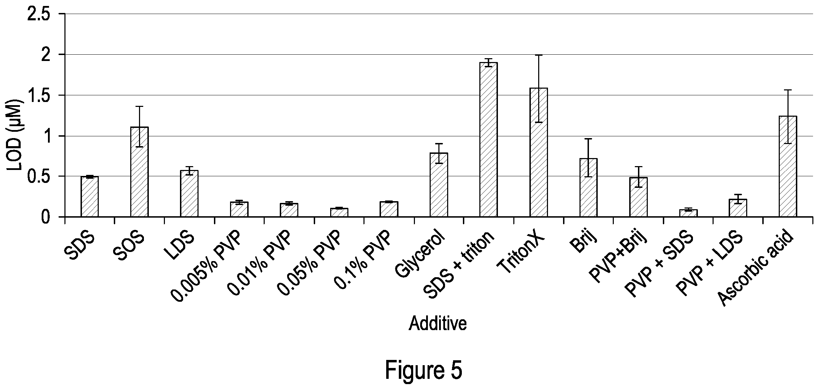

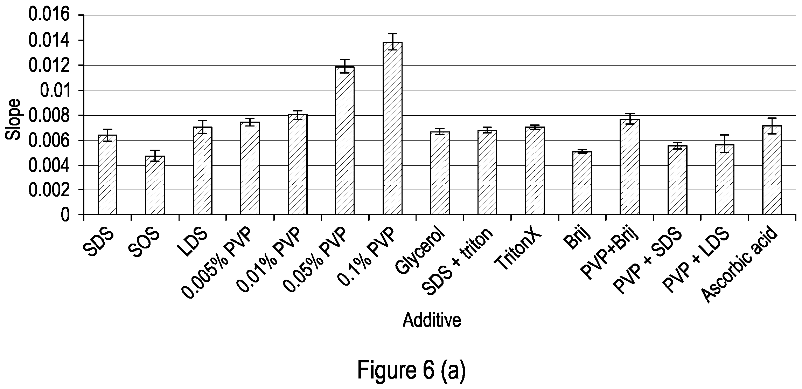

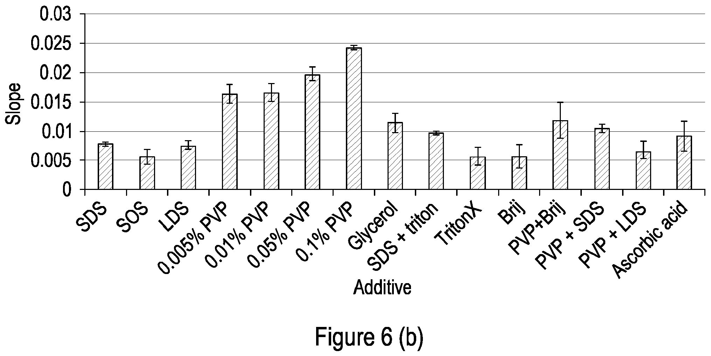

Figures 5 and 6 show the limits of detection calibration slopes for each of the additives trialled. It can be seen that the PVP additions show an improved sensitivity (calibration slope) and stability (demonstrable from Figure 5) relative to all other additives tested. This results in a limit of detection at least 1 order of magnitude less than all other additives. From Figure 6, it may be seen that the PVP increases the sensitivity for lower phosphate concentrations. This is particularly useful for environmental samples which are generally < 2µM, and open ocean samples which are frequently found at nano-molar concentrations.

### Interferences

The molybdenum blue assay is also used to measure arsenate and silicate. These are therefore common interferences that have been included in this study. Changes in optical properties with increasing salinity means that is important to quantify the impact of ionic strength on the assay.

### Silicate

The tables below show the response of the assay for each additive with silicate. It is unlikely that silicate would be found in natural systems above 200 µM. However it is useful to show interferences above natural concentrations and demonstrate the limitations of a system.

The table below shows the apparent phosphate concentrations in simplified artificial seawater (ASW) with increasing silicate additions (at 0 µM phosphate). When PVP is employed as the only additive, the apparent phosphate concentration is <0 i.e. there is no interference from silica. When PVP is employed with a surfactant, there is interference.

| **Additive** | **Wavelength (nm)** | **Apparent PO₄ (µM) concentration** | | |
|---|---|---|---|---|
| | | **100 µM SiO₄** | **200 µM SiO₄** | **400 µM SiO₄** |
| SDS | 880 | 0.37 +/- 0.31 | 0.88 +/- 0.59 | 2.14 +/- 0.67 |
| SOS | 880 | <0 | <0 | <0 |
| LDS | 880 | <0 | 0.29 +/- 0.09 | 0.83 +/- 0.75 |
| 0.005% PVP | 720 | <0 | <0 | <0 |
| 0.01% PVP | 725 | <0 | <0 | <0 |
| 0.05% PVP | 730 | <0 | <0 | <0 |
| 0.1 % PVP | 730 | <0 | <0 | <0 |
| Glycerol | 880 | <0 | 0.15 +/-0.09 | 0.02 +/-0.05 |
| Triton X | 700 | <0 | <0 | <0 |
| Brij 35 | 700 | <0 | <0 | 0.59 +/- 0.45 |
| 0.01 % PVP + Brij 35 | 715 | 0.04 +/-0.06 | 0.04 +/-0.03 | 0.51 +/-0.45 |
| 0.01% PVP + SDS | 725 | 0.60 +/-0.10 | 0.53 +/-0.10 | 1.21 +/-0.92 |
| 0.01% PVP + LDS | 725 | 0.57 +/-0.20 | 0.57 +/-0.13 | 0.64 +/-0.12 |
| SDS + Triton X | 880 | 0.10 +/-0.04 | 0.17 +/-0.04 | 0.94 +/-1.05 |

The table below shows the apparent phosphate concentrations in milli-q (MQ) water with increasing silicate additions (at 0 µM phosphate). When PVP is employed as the only additive at a concentration of 0.1% PVP, the apparent phosphate concentration is <0 for 100 and 200µM silicate i.e. there is no interference from silica under these conditions. However, when PVP is employed at lower concentrations in MQ there is interference from silica.

| **Additive** | **Wavelength (nm)** | **Apparent PO₄ (µM) concentration** | | |
|---|---|---|---|---|
| | | **100 µM SiO₄** | **200 µM SiO₄** | **400 µM SiO₄** |
| None | 880 | <0 | <0 | <0 |
| SDS | 880 | 0.30 +/-0.05 | 0.42 +/-0.07 | 0.40 +/- 0.26 |
| SOS | 880 | <0 | <0 | <0 |
| LDS | 880 | 0.31 +/-0.14 | 0.59 +/-0.08 | 1.07 +/- 0.68 |
| 0.005% PVP | 720 | 0.13 +/-0.04 | 0.17 +/-0.05 | 0.13 +/- 0.04 |
| 0.01% PVP | 725 | 0.10 +/-0.02 | 0.14 +/-0.01 | 0.10 +/- 0.02 |
| 0.05% PVP | 730 | 0.94 +/-0.04 | 0.03 +/-0.04 | 0.09 +/- 0.04 |
| 0.1 % PVP | 730 | <0 | <0 | 0.05 +/-0.02 |
| Glycerol | 880 | <0 | <0 | <0 |
| Triton X | 700 | 0.96 +/-0.13 | 1.43 +/-0.07 | 1.46 +/- 0.16 |
| Brij 35 | 700 | <0 | <0 | <0 |
| 0.01 % PVP + Brij 35 | 715 | 0.24 +/-0.06 | 0.32 +/-0.04 | 0.29 +/- 0.02 |
| 0.01% PVP + SDS | 725 | 1.18 +/-0.28 | 1.33 +/-0.04 | 1.54 +/- 0.15 |
| 0.01% PVP + LDS | 725 | <0 | 0.46 +/-0.08 | 0.59 +/- 0.09 |
| SDS + Triton X | 880 | 3.97 +/-0.32 | 3.49 +/-0.39 | 3.67 +/- 0.26 |

Comparing the results for ASW and MQ, it can be seen that there is less interference in ASW (or higher ionic strength water). All PVP additions in ASW effectively suppressed any silicate interferences. In MQ medium there is approximately 100 nM apparent phosphate with 200 µM silicate.

### Arsenate

The arsenic drinking water limit as set by the U.K. Drinking Water Inspectorate and World Health Organisation is 10 µg L⁻¹ As; this equates to ~ 0.1 µM As. The table below shows the "apparent phosphate" concentrations. This is the contribution to the phosphate signal that may be present at certain As concentrations. The apparent phosphate increases with no additive (ascorbic acid only) until 1 µM As, but produces no interference <0.1 µM As; SDS and Brij^{®} produce increasing apparent phosphate with 0.5µM As, and then a decrease at 1 µM (this may be due to insufficient reaction times for the As); SOS, Glycerol and PVP mixed with Brij^{®} all give increasing apparent phosphate signals with increasing As concentrations; Triton X produced no interference, however this is most likely a result of a raised and unstable baseline (calibration intercept); and the LDS produced apparent phosphate at even low As concentrations (0.05 µM). Only the lower PVP concentrations showed any interference from As, with the phosphate signal enhanced by up -30% in the presence of 1 µM As (which is only likely in contaminated ground-waters).

| **Additive** | **Wavelength (nm)** | **Apparent PO₄ (µM) concentration** | | | |
|---|---|---|---|---|---|
| | | **0.05 µM As** | **0.1 µM As** | **0.5 µM As** | **1 µM As** |
| None | 880 | <0 | 0.10 +/-0.12 | 0.10 +/-0.01 | 0.33 +/-0.23 |
| SDS | 880 | <0 | 0.12 +/-0.01 | 0.52 +/-0.02 | 0.45 +/-0.01 |
| SOS | 880 | <0 | 0.10 +/-0.01 | 0.13 +/-0.01 | 0.19 +/-0.02 |
| LDS | 880 | 0.034 +/-0.01 | 0.25 +/-0.02 | 0.49 +/-0.04 | 0.28 +/-0.08 |
| 0.005% PVP | 720 | <0 | <0 | 0.13 +/-0.03 | 0.26 +/-0.04 |
| 0.01% PVP | 725 | <0 | <0 | <0 | 0.27 +/-0.01 |
| 0.05% PVP | 730 | <0 | <0 | <0 | 0.09 +/-0.01 |
| 0.1 % PVP | 730 | <0 | <0 | <0 | <0 |
| Glycerol | 880 | <0 | <0 | 0.01 +/-0.002 | 0.37 +/-0.46 |
| Triton X | 700 | <0 | <0 | <0 | <0 |
| Brij 35 | 700 | <0 | 0.20 +/-0.01 | 0.62 +/-0.07 | 0.22 +/-0.02 |
| 0.01 % PVP + Brij 35 | 715 | <0 | 0.07 +/-0.002 | 0.42 +/-0.004 | 0.76 +/-0.04 |
| 0.01% PVP + SDS | 725 | <0 | <0 | <0 | <0 |
| 0.01% PVP + | 725 | <0 | 0.24 +/-0.01 | 0.03 +/-0.004 | 0.39 +/-0.04 |
| SDS + Triton X | | <0 | <0 | <0 | <0 |

### Matrix effect

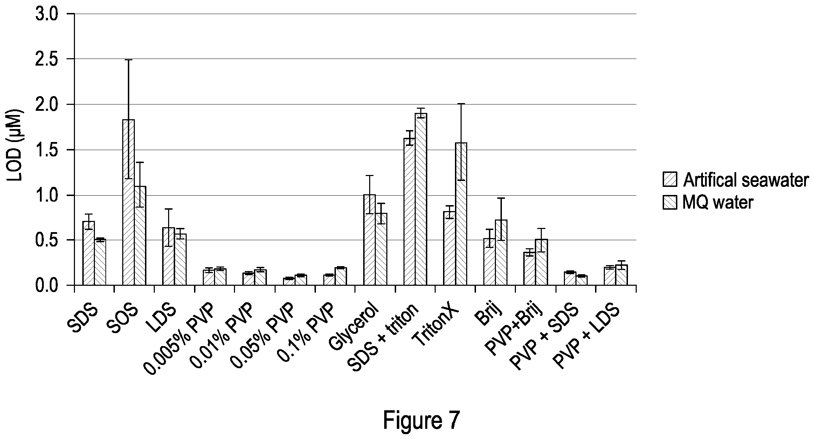

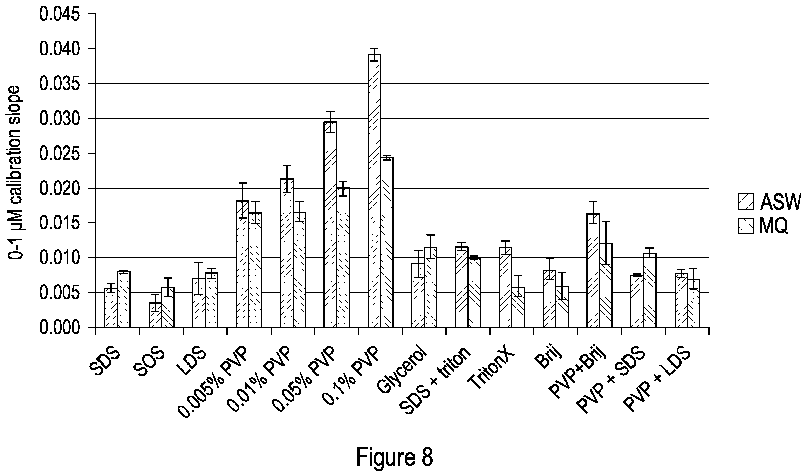

Figures 7 and 8 show the impact of ionic strength of the sample solution on the limits of detection and sensitivity of the assay. The limit for detection is lower for ASW than for MQ when PVP is employed alone. The difference between the ASW and MQ limits may be small, but due to the low error (high precision) these differences are significant (P<0.01). It can also be seen from Figure 8 that with increasing PVP concentrations and ionic strength, the sensitivity of the assay increases.

### Kinetics

### Methodology

An Aglient Cary-60 UV-visible spectrophotometer was used to measure the absorbance at the peak wavelengths detailed in the table above. A SFA-20 rapid kinetics accessory (Hi-Tech, TgK Scientific) was used with 2 mL pneumatically driven syringes to enhance mixing consistency. The optical path length of the quartz cuvette was 1 cm, and the system was temperature controlled by flowing water from a water bath through the flow circuit around the accessory. Only the data from 20°C is presented here. Measurements were taken every 10 seconds for up to 15 minutes, and repeated 4 times. A 0.1M NaOH flush solution was used when a reagent or sample was changed to ensure no carryover. Colour development for phosphate concentrations of 0, 1, 5 and 10 µM were measured.

Here only the PVP and SDS have been characterised and compared with ascorbic acid alone. This is because the SDS is the most commonly used surfactant, and the PVP from the microplate scans far outperformed all other additives. All kinetic data has been normalised to a starting point (t=0) of AU =0 so that end points are comparable (difference between the blank and standard).

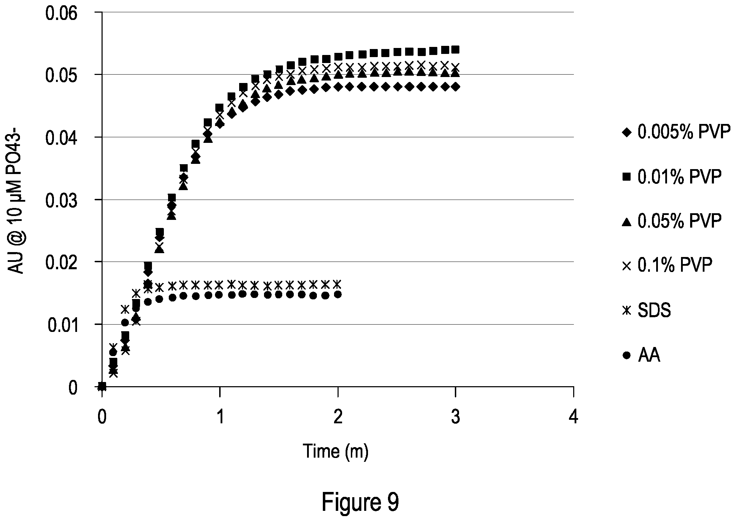

Figure 1 shows colour formation (absorption units) as measured at the peak absorbance wavelength for each additive. The PVP induces an autocatalytic rate reaction, evident from the sinusoidal curve in Figure 9. The SDS and AA alone produce a straight forward 1^{st} order rate which confers the reaction mechanism as below:

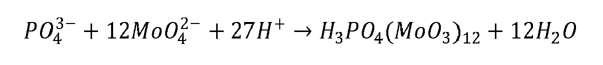

Reduced to mixed valence spp with spectral absorbance peaks at 700 and 880 nm

*H₃PMo*(*VI*)₁₂*O*₄₀ + 4*e⁻* → [*H*₄*PMo*(*VI*)₈*Mo*(*V*)₄*O*₄₀]³⁻

The sinusoidal curve present when PVP is added to the ascorbic acid suggests that it is also taking part in the reaction. What we see initially is a slower reaction rate, which after about 30 seconds speeds up as more reactant/product is produced. We propose that this could be due to a number of reasons:
1. The PVP could initially act as a dispersant, thereby reducing initial collisions. This dispersant is useful in that it prevents any of the phosphomolybdic acid precipitating, as it is less soluble in its reduced from.
2. The PVP could be acting as a reducing agent accelerating the reaction.
3. The increased absorbance with the PVP addition could indicate an alternative compound formed with the phosphomolybdic acid and PVP.

### Microfluidic systems

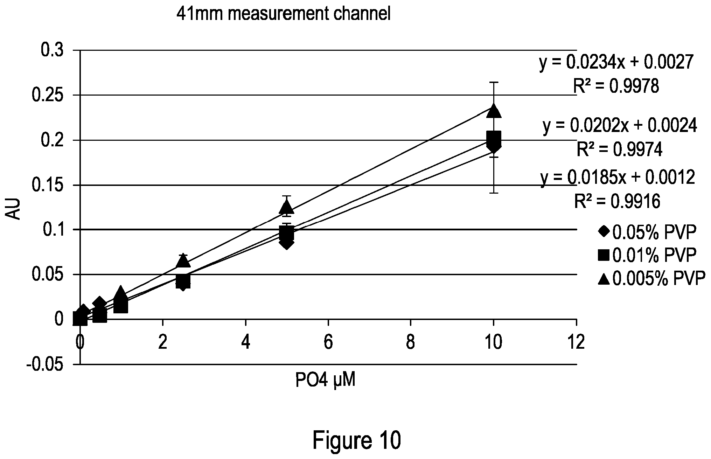

SDS was the original surfactant used with this system but the high Krafft point (~15°C) made it un-suited to an in situ device. Removing the surfactant entirely results in a loss of sensitivity when the phosphate concentration <1 µM, and affects the precision of the system. Figure 10 shows a calibration curve for a 41mm measurement cell in a microfluidic device. This is to determine both linearity of the calibration, and sensitivity (slope of the calibration curve). Referring to figure 10 it can be seen that there is little difference between the 0.005% and 0.01% PVP additions.

## Claims

1. A process for measuring a concentration of a chemical species in an aqueous sample comprising:
providing an aqueous sample containing the chemical species, the sample being obtained from industrial water or agricultural water or waste water or water for aquaculture or a body of water selected from an ocean, a sea, an estuary, a river, a reservoir, a lake, a ditch or a pond;
mixing the aqueous sample with a first reagent that comprises a solution of a molybdenum VI salt to provide a first solution;
mixing the first solution with a second reagent that comprises a reducing agent to form a second solution; and
measuring a property of the second solution to determine the concentration of the chemical species;
wherein polyvinylpyrrolidone is added to the aqueous sample, the first reagent, the second reagent, the first solution and/or the second solution such that the second solution comprises no more than 0.5% (w/v) polyvinylpyrrolidone.

2. The process of claim 1, wherein the polyvinylpyrrolidone is added to the first reagent.

3. The process of claim 2, wherein the first reagent comprises no more than 1% (w/v) polyvinylpyrrolidone.

4. The process of claim 1, wherein the polyvinylpyrrolidone is added to the second reagent.

5. The process of claim 4, wherein the second reagent comprises no more than 1% (w/v) polyvinylpyrrolidone.

6. The process of any one of the preceding claims, wherein the mass of polyvinylpyrrolidone corresponds to at least 10% of the mass of the molybdenum VI salt.

7. The process of any one of the preceding claims, wherein the chemical species is selected from (i) phosphate, (ii) arsenate or (iii) germanate.

8. The process of any one of the preceding claims, wherein the aqueous sample is saline.

9. The process of any one of the preceding claims, wherein
(i) the molybdenum VI salt comprises ammonium molybdate and / or sodium molybdate; and/or
(ii) the reducing agent is ascorbic acid or tin chloride.

10. The process of any one of the preceding claims, wherein measuring the property of the second solution comprises measuring the intensity of light that is transmitted or absorbed by the second solution.

11. The process of any one of the preceding claims, wherein (i) the first solution is a yellow solution and/or (ii) the second solution is a blue solution.

12. The process of any one of the preceding claims, comprising an initial step of oxidizing the aqueous sample.

13. The process of any one of the preceding claims, wherein the second solution has a volume of no more than 100ml, preferably no more than 1ml.

14. The process of any one of the preceding claims, which is carried out on a lab-on-chip device.

15. The process of any one of the preceding claims, which is carried out on an analyser selected from a micro-fluidic sensor, a stopped flow analyser, a segmented flow analyser, a flow injection analyser, or a continuous flow analyser.

## Patentansprüche

1. Prozess zum Messen einer Konzentration einer chemischen Spezies in einer wässrigen Probe, umfassend:
Bereitstellen einer wässrigen Probe, die die chemische Spezies enthält, wobei die Probe aus Industriebrauchwasser oder landwirtschaftlichem Wasser oder Abwasser oder Wasser für Aquakultur oder einem Gewässer, ausgewählt aus einem Ozean, einem Meer, einer Flussmündung, einem Fluss, einem Reservoir, einem See, einem Graben oder einem Teich, erhalten wird;
Mischen der wässrigen Probe mit einem ersten Reagens, das eine Lösung eines Molybdän-VI-Salzes umfasst, um eine erste Lösung bereitzustellen;
Mischen der ersten Lösung mit einem zweiten Reagens, das ein Reduktionsmittel umfasst, um eine zweite Lösung zu bilden; und
Messen einer Eigenschaft der zweiten Lösung, um die Konzentration der chemischen Spezies zu bestimmen;
wobei Polyvinylpyrrolidon zu der wässrigen Probe, dem ersten Reagens, dem zweiten Reagens, der ersten Lösung und/oder der zweiten Lösung derart beigegeben wird, dass die zweite Lösung nicht mehr als 0,5 % (Gew./Vol.) Polyvinylpyrrolidon umfasst.

2. Prozess nach Anspruch 1, wobei das Polyvinylpyrrolidon dem ersten Reagens zugegeben wird.

3. Prozess nach Anspruch 2, wobei das erste Reagens nicht mehr als 1 % (Gew./Vol.) Polyvinylpyrrolidon umfasst.

4. Prozess nach Anspruch 1, wobei das Polyvinylpyrrolidon dem zweiten Reagens zugegeben wird.

5. Prozess nach Anspruch 4, wobei das zweite Reagens nicht mehr als 1 % (Gew./Vol.) Polyvinylpyrrolidon umfasst.

6. Prozess nach einem der vorstehenden Ansprüche, wobei die Masse von Polyvinylpyrrolidon mindestens 10 % der Masse des Molybdän-VI-Salzes entspricht.

7. Prozess nach einem der vorstehenden Ansprüche, wobei die chemische Spezies aus (i) Phosphat, (ii) Arsenat oder (iii) Germanat ausgewählt wird.

8. Prozess nach einem der vorstehenden Ansprüche, wobei die wässrige Probe salzhaltig ist.

9. Prozess nach einem der vorstehenden Ansprüche, wobei
(i) das Molybdän-VI-Salz Ammoniummolybdat und / oder Natriummolybdat umfasst; und/oder
(ii) das Reduktionsmittel Ascorbinsäure oder Zinnchlorid ist.

10. Prozess nach einem der vorstehenden Ansprüche, wobei Messen der Eigenschaft der zweiten Lösung Messen der Intensität eines von der zweiten Lösung durchgelassenen oder absorbierten Lichts umfasst.

11. Prozess nach einem der vorstehenden Ansprüche, wobei (i) die erste Lösung eine gelbe Lösung ist und/oder (ii) die zweite Lösung eine blaue Lösung ist.

12. Prozess nach einem der vorstehenden Ansprüche, der einen Anfangsschritt zum Oxidieren der wässrigen Probe umfasst.

13. Prozess nach einem der vorstehenden Ansprüche, wobei die zweite Lösung ein Volumen von nicht mehr als 100 ml, bevorzugt nicht mehr als 1 ml aufweist.

14. Prozess nach einem der vorstehenden Ansprüche, der auf einer Lab-on-Chip-Vorrichtung durchgeführt wird.

15. Prozess nach einem der vorstehenden Ansprüche, der auf einem Analysator durchgeführt wird, der aus einem Mikrofluidsensor, einem Analysator mit gestopptem Durchfluss, einem Analysator mit segmentiertem Durchfluss, einem Durchflussinjektionsanalysator oder einem Analysator mit kontinuierlichen Durchfluss ausgewählt ist.

## Revendications

1. Processus de mesure d'une concentration d'une espèce chimique dans un échantillon aqueux, comprenant :
la fourniture d'un échantillon aqueux contenant l'espèce chimique, l'échantillon étant obtenu à partir d'eau industrielle ou d'eau agricole ou d'eaux usées ou d'eau pour l'aquaculture ou d'une masse d'eau sélectionnée parmi un océan, une mer, un estuaire, une rivière, un réservoir, un lac, un fossé ou un étang ;
le mélange de l'échantillon aqueux avec un premier réactif qui comprend une solution d'un sel de molybdène VI pour obtenir une première solution ;
le mélange de la première solution avec un second réactif qui comprend un agent réducteur pour former une seconde solution ; et
la mesure d'une propriété de la seconde solution pour déterminer la concentration de l'espèce chimique ;
dans lequel de la polyvinylpyrrolidone est ajoutée à l'échantillon aqueux, au premier réactif, au second réactif, à la première solution et/ou à la seconde solution de sorte que la seconde solution ne comprenne pas plus de 0,5 % (p/v) de polyvinylpyrrolidone.

2. Processus selon la revendication 1, dans lequel la polyvinylpyrrolidone est ajoutée au premier réactif.

3. Processus selon la revendication 2, dans lequel le premier réactif ne comprend pas plus de 1 % (p/v) de polyvinylpyrrolidone.

4. Processus selon la revendication 1, dans lequel la polyvinylpyrrolidone est ajoutée au second réactif.

5. Processus selon la revendication 4, dans lequel le second réactif ne comprend pas plus de 1 % (p/v) de polyvinylpyrrolidone.

6. Processus selon l'une quelconque des revendications précédentes, dans lequel la masse de polyvinylpyrrolidone correspond à au moins 10% de la masse du sel de molybdène VI.

7. Processus selon l'une quelconque des revendications précédentes, dans lequel l'espèce chimique est sélectionnée parmi (i) du phosphate, (ii) de l'arséniate ou (iii) du germanate.

8. Processus selon l'une quelconque des revendications précédentes, dans lequel l'échantillon aqueux est salin.

9. Processus selon l'une quelconque des revendications précédentes, dans lequel
(i) le sel de molybdène VI comprend du molybdate d'ammonium et/ou du molybdate de sodium ; et/ou
(ii) l'agent réducteur est de l'acide ascorbique ou du chlorure d'étain.

10. Processus selon l'une quelconque des revendications précédentes, dans lequel la mesure de la propriété de la seconde solution comprend la mesure de l'intensité de la lumière qui est transmise ou absorbée par la seconde solution.

11. Processus selon l'une quelconque des revendications précédentes, dans lequel (i) la première solution est une solution jaune et/ou (ii) la seconde solution est une solution bleue.

12. Processus selon l'une quelconque des revendications précédentes, comprenant une étape initiale d'oxydation de l'échantillon aqueux.

13. Processus selon l'une quelconque des revendications précédentes, dans lequel la seconde solution présente un volume ne dépassant pas 100 ml, de préférence ne dépassant pas 1 ml.

14. Processus selon l'une quelconque des revendications précédentes, qui est réalisé sur un dispositif laboratoire sur puce.

15. Processus selon l'une quelconque des revendications précédentes, qui est réalisé sur un analyseur sélectionné parmi un capteur microfluidique, un analyseur à flux interrompu, un analyseur à flux segmenté, un analyseur à injection de flux ou un analyseur à flux continu.
